(19) Europäisches Patentamt
European Patent Office
Office européen des brevets

(11) Veröffentlichungsnummer: 0 201 732
A2

(12) **EUROPÄISCHE PATENTANMELDUNG**

(21) Anmeldenummer: **86104951.8**

(22) Anmeldetag: **10.04.86**

(51) Int. Cl.⁴: **A61K 31/135**, A61K 31/275, A61K 31/165

(30) Priorität: **24.04.85 DE 3514725**

(43) Veröffentlichungstag der Anmeldung: **20.11.86 Patentblatt 86/47**

(84) Benannte Vertragsstaaten: **BE CH DE FR GB IT LI LU NL SE**

(71) Anmelder: **BOEHRINGER INGELHEIM VETMEDICA GMBH**

**D-6507 Ingelheim/Rhein(DE)**

(72) Erfinder: **Salamon, Ernst, Dr.**
**Kurpfalzstrasse 8**
**D-6507 Ingelheim(DE)**
Erfinder: **Briganti, Alberto, Dr.**
**Via Petrarca 33**
**Figline Valdarno I-50 100 Firenze(IT)**

(74) Vertreter: **Laudien, Dieter, Dr.**
**Boehringer Ingelheim Zentrale GmbH ZA**
**Patente Postfach 200**
**D-6507 Ingelheim am Rhein(DE)**

(54) **Beta2-Mimetica vom Typ der 4-Amino-phenyläthanolamine zur Verhinderung von peptischen Magen-Geschwüren.**

(57) Die Erfindung betrifft die neue Verwendung von $\beta_2$-Mimetica vom Typ der 4-Amino-phenyläthanolamine der allgemeinen Formel

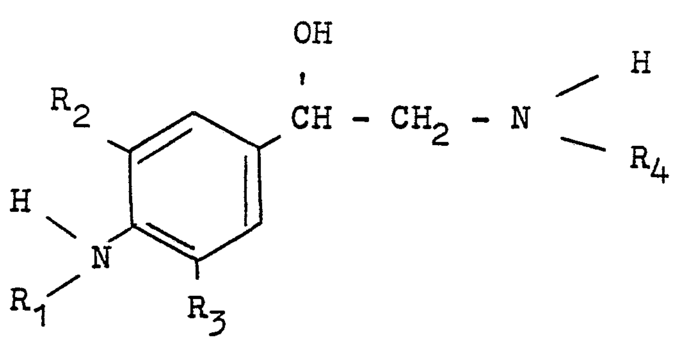

,(I)

in der

$R_1$ ein Wasserstoffatom oder eine Alkoxycarbonylgruppe mit insgesamt 2 bis 5 Kohlenstoffatomen,

$R_2$ ein Wasserstoff-, Fluor-oder Chloratom,

$R_3$ ein Chlor-oder Bromatom, eine Cyan-oder Trifluormethylgruppe und

$R_4$ eine verzweigte Alkylgruppe mit 3 bis 5 Kohlenstoffatomen oder eine Cycloalkylgruppe mit 3 bis 5 Kohlenstoffatomen bedeuten, und deren physiologi-

EP 0 201 732 A2

sch verträgliche Säureadditionssalze zur Verhinderung von peptischen Ulcera bei der Protein-Masternährung.

$\beta_2$-Mimetica vom Typ der 4-Amino-phenyläthanolamine zur Verhinderung von peptischen Magen-Geschwüren

In den US-Patentschriften 3.536.712, 4.063.025, 4.119.710 und 4.214.001 werden $\beta_2$-Mimetica vom Typ der 4-Amino-phenyläthanolamine der allgemeinen Formel

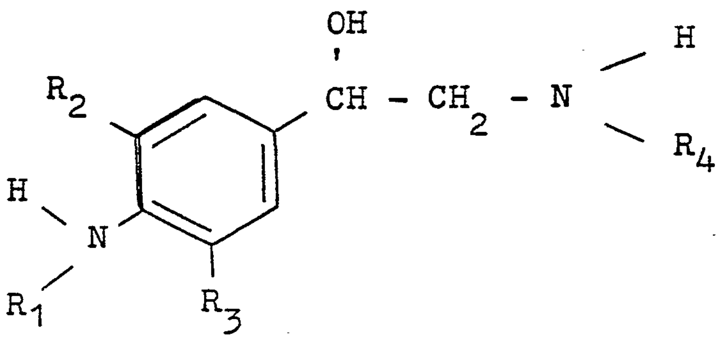
,(I)

und deren physiologisch verträgliche Säureadditionssalze beschrieben, welch eine sehr gute orale Resorption aufweisen.

In der obigen allgemeinen Formel I bedeutet

$R_1$ ein Wasserstoffatom oder eine Alkoxycarbonylgruppe mit insgesamt 2 bis 5 Kohlenstoffatomen,

$R_2$ ein Wasserstoff-, Fluor-oder Chloratom,

$R_3$ ein Chlor-oder Bromatom, eine Cyan-oder Trifluormethylgruppe und

$R_4$ eine verzweigte Alkylgruppe mite 3 bis 5 Kohlenstoffatomen oder eine Cycloalkylgruppe mit 3 bis 5 Kohlenstoffatomen.

Für die bei der Definition der Reste $R_1$ und $R_4$ erwähnten Bedeutungen kommen beispielsweise

für $R_1$ die Bedeutung des Wasserstoffatoms, der Methoxycarbonyl-, Äthoxycarbonyl-, n-Propoxycarbonyl-, Isopropoxycarbonyl-, n-Butoxycarbonyl-oder Isobutoxycarbonylgruppe und

für $R_4$ die der Isopropyl-, Isobutyl-, tert.Butyl-, Isoamyl-, Neopentyl-, tert.Pentyl-, Cyclopropyl-, Cyclobutyl-oder Cyclopentylgruppe in Betracht.

Desweiteren ist bekannt, daß die wirksamsten Vertreter dieser $\beta_2$-Mimetica, z.B. 1-(4-Amino-3,5-dichlor-phenyl)-2-tert.butylamino-äthanol (siehe Arzneimittelforschung 26, 1404-1420 (1976)), bei Untersuchungen an der Ratte mit um den Faktor 100 höheren Dosen, als sie zur Erzielung einer therapeutisch ausnutzbaren broncholytischen Wirkung erforderlich sind, eine Hemmwirkung auf die Magensäuresekretion bei Ratten aufweisen.

Außerdem ist aus der EP-A-0.057.900 bekannt, daß bei Verwendung einer Verbindung der obigen allgemeinen Formel I und deren verträglichen Säureadditionssalzen als Futterzusatz bei der Tierhaltung eine Verbesserung der Futterverwertung, des Futterverbrauchs, der Gewichtszunahme und eine Qualitätsverbesserung der Schlachtkörper, insbesondere jedoch eine Verbesserung des Muskel/Fett-Verhältnisses erzielt wird.

Überraschenderweise wurde nun gefunden, daß bei Verwendung einer Verbindung der obigen allgemeinen Formel I und deren physiologisch verträglichen Säureadditionssalzen als Futterzusatz bei der Protein-Masternährung, insbesondere von Kälbern mit Milch mit erhöhtem Proteingehalt, peptische Ulcera, insbesondere peptische Labmagenulcera, verhindert werden. Labmagengeschwüre der Mastkälber sind peptische Geschwüre, als deren Ursache Streß, der nicht weiter definiert ist, gilt.

Hierzu wird erfindungsgemäße eine obige Verbindung dem Masttier, insbesondere Kälbern, in einer Tagesdosis von 1 bis 10 mcg/kg Körpergewicht, vorzugsweise jedoch von 1,5, bis 5 mcg/kg Körpergewicht oral und dem Futter beigemischt, verabreicht.

Hierbei haben sich die Verbindungen der allgemeinen Formel I, in der

$R_1$ ein Wasserstoffatom oder eine Äthoxycarbonylgruppe,

$R_2$ ein Fluor-oder Chloratom,

$R_3$ ein Chloratom, eine Cyan-oder Trifluormethylgruppe und

$R_4$ eine verzweigte Alkylgruppe mit 3 bis 5 Kohlenstoffatomen bedeuten, insbesondere jedoch die Verbindungen

1-(4-Amino-3,5-dichlor-phenyl)-2-tert.butylamino-äthanol,

1-(4-Amino-3-chlor-5-trifluomethyl-phenyl)-2-tert.butylamino-äthanol und

1-(4-Äthoxycarbonylamino-3-cyan-5-fluor-phenyl)-2-tert.butylamino-äthanol

sowie deren physiologisch verträgliche Säureadditionssalze als besonders geeignet zur Verhinderung von peptischen Geschwüren, insbesondere von Labmagenulcera bei Kälbern, erwiesen.

Beispielsweise wurde die Verbindung 1-(4-Amino-3,5,-dichlorphenyl)-2-tert.butylamino-äthanol-hydrochlorid in einer Tagesdosis von 2 mcg/kg Körpergewicht, verteilt auf 2 Dosen, welche dem Futter beigemischt war, 32 bei Beginn der Behandlung ca. 70 Tage alten und ca. 100 kg schweren Kälbern im Vergleich zu einer gleichgroßen Kontrollgruppe fortgesetzt verabreicht.

14 Tage vor dem Schlachttermin wurde die Behandlung beendet.

Nach der Schlachtung zeigten sich bei der Sektion der Labmägen der Kontrollgruppe Läsionen der Labmagenschleimhaut in folgenden verschiedenen Stadien:

3 Tiere hatten je ein Ulcus bis zu einem Durchmesser von 20 mm

4 Tiere hatten mehrere Ulcera mit einem Durchmesser von 2 bis 15 mm

Weitere Tiere wiesen hämorrhagische Erosionen auf den Magenschleimhautfalten auf.

Demgegenüber war bei keinem Tier der Versuchsgruppe irgendwelche Läsionen der Labmagenschleimhaut feststellbar.

Außerdem wies bei einem Futterverbrauch der Versuchsgruppe von 344,3 kg pro Tier bzw. von 343,7 kg pro Tier der Kontrollgruppe die erstere eine um ca. 10 kg höhere Endgewicht pro Tier auf. Bei einem annähernd gleichen Futterverbrauch pro Tier bedeutet dies eine Steigerung der Fleischproduktion von ca. 320 kg in der Versuchsgruppe.

Die verabreichte Substanz ist gut verträglich. Diese weist beispielsweise eine $LD_{50}$ an der Maus von 176 mg/kg p.o. auf. Ferner traten bei der Applikation dieser Substanz keinerlei Nebenwirkungen auf, so ergaben sich beispielsweise bei der hämatologischen Untersuchung und der Enzyme SGOT, CPK, LDH und GLDH, welche stichprobenartig erfolgte, zwischen beiden Gruppen keine signifikanten Unterschiede.

Die obigen günstigen Ergebnisse konnten nicht vorhergesehen werden, da

1. die untersuchte Substanz in der für die broncholytische bzw. tokolytische Wirkung erforderlichen terapeutischen Dosis angwendet wurde und

2. ein Behandlungsversuch mit dem Magensäuresekretionshemmer Cimetidine erfolglos blieb.

Die obigen Befunde, insbesondre im Vergleich mit Cimetidine, zeigen daher, daß die an der Ratte bei Versuchen zur Magensäuresekretionshemmung mit weit höheren Dosen erzielten Ergebnisse nicht primär auf das Kalb übertragen werden können.

Die erfindungsgemäße Applikation einer Verbindungen der obigen allgemeinen Formel I erhöht somit in nicht vorhersehbarer Weise die Toleranz des Kälbermagens gegenüber der dem Alter und der Größe der Tiere inadäquaten bzw. unphysiologischen Futterzusammensetzung. Sie vermeidet damit die bisher unvermeidlichen Verluste bei der intensiven Mast von Weißfleischkälbern aller dazu verwandten Rinderrassen (insbesondere der Braunvieh-, Fleckvieh-, Charolais-, Schwarzbunt-, Rotbunt-und Anglerrasse), die über ca. 5 Monate bis zu einem Gewicht von ca. 250 kg gemästet werden, durch Mortalität infolge Ulcera und durch schlechte Tageszunahmen infolge peptischer Labmagenulcera.

Die nachfolgenden Beispiele sollen die Erfindung näher erläutern:

Beispiel A

1-(4-Äthoxycarbonylamino-3-chlor-5-fluor-phenyl)-2-tert.butylamino-äthanol-hydrochlorid

In eine Lösung von 3,7 g Selendioxid in 30 ml Dioxan und 1 ml Wasser werden bei 60°C unter Rühren 8,7 g 4'-Äthoxycarbonylamino-3'-chlor-5'-fluor-acetophenon portionsweise eingetragen. Anschließend wird 4 Stunden lang auf Rückflußtemperatur erhitzt. Zu der so dargestellten Lösung von 4'-Äthoxycarbonylamino-3'-chlor-5'-fluor-phenylglykol werden nach Abkühlen und unter Außenkühlung mit Eis 4,1 ml tert.Butylamin getropft. Nach beendeter Zugabe verdünnt man mit 360 ml Äthanol und filtriert vom Ungelösten ab. Die

das rohe 4'-Äthoxycarbonylamino-3'-chlor-5'-fluor-phenyl-glyoxyliden-tert.butylamin enthaltende Lösung wird unter Rühren und Kühlen mit Eis portionsweise mit 5 g Natriumborhydrid versetzt und über Nacht bei Raumtemperatur stehen gelassen. Anschliessend zerstört man überschüssiges Natriumborhydrid mit Aceton, versetzt mit Wasser und extrahiert mit Chloroform. Die Chloroform-Lösung wird mit Wasser gewaschen, mit Natriumsulfat getrocknet, nach Zusatz von Aktivkohle kurz aufgekocht, filtriert und im Vakuum zur Trockne eingedampft. Der feste Rückstand von 1-(4-Äthoxycarbonyl-amino-3-chlor-5-fluor-phenyl)-tert.butylamino-äthanol wird in Isopropanol aufgenommen und mit ätherischer Salzsäure bis pH 4 angesäuert. Nach Zugabe von Äther tritt Kristallisation ein. Die Kristalle werden abgesaugt und mit Äther gewaschen.

Schmelzpunkt: 192-193°C.

Analog Beispiel A werden folgende Verbindungen hergestellt:

1-(4-Äthoxycarbonylamino-3-chlor-5-trifluormethyl-phenyl)-2-tert.butylamino-äthanol

Schmelzpunkt: 168-170°C (Zers.)

1-(4-Äthoxycarbonylamino-3-brom-5-fluor-phenyl)-2-isopropylamino-äthanol-hydrochlorid

Schmelzpunkt: 180-182°C

1-(4-Äthoxycarbonylamino-3-brom-5-fluor-phenyl)-2-tert.butylamino-äthanol-hydrochlorid

Schmelzpunkt: 197-198°C

1-(4-Äthoxycarbonylamino-3-cyan-5-fluor-phenyl)-2-tert.butylamino-äthanol-hydrochlorid

Schmelzpunkt: 198-200°C

1-(3-Cyan-5-fluor-4-isobutyloxycarbonylamino-phenyl)-2-tert.butylamino-äthanol-hydrochlorid

(Das überschüssige Natriumborhydrid wurde mit verdünnter Salzsäure zerstört)

Schmelzpunkt: 189-191°C

1-(4-Äthoxycarbonylamino-3-cyan-phenyl)-2-isopropylamino-äthanol

Schmelzpunkt: 112-115°C

1-(4-Äthoxycarbonylamino-3-cyan-phenyl)-2-tert.butylamino-äthanol

Schmelzpunkt: 78-82°C

1-(4-Amino-3-chlor-5-cyan-phenyl)-2-tert.butylamino-äthanol

Schmelzpunkt: 131-135°C

1-(4-Amino-3-chlor-5-fluor-phenyl)-2-isopropylamino-äthanolhydrochlorid

Schmelzpunkt: 152-154°C (Zers.)

1-(4-Amino-3-chlor-5-fluor-phenyl)-2-cylcopropylamino-äthanol hydrochlorid

Schmelzpunkt: 175-177°C (Zers.)

1-(4-Amino-3-chlor-5-fluor-phenyl)-2-tert.butylamino-äthanol-hydrochlorid

Schmelzpunkt: 206-208°C (Zers.)

1-(4-Amino-3-chlor-5-fluor-phenyl)-2-tert.pentylamino-äthanol-hydrochlorid

Schmelzpunkt: 187-188°C (Zers.)

1-(4-Amino-3-chlor-5-fluor-phenyl)-2-isopropylamino-äthanolhydrochlorid

Schmelzpunkt: 171-173°C (Zers.)

1-(4-Amino-3-brom-5-fluor-phenyl)-2-tert.butylamino-äthanol-hydrochlorid

Schmelzpunkt: 207-208°C (Zers.)

1-(4-Amino-3-brom-5-fluor-phenyl)-2-cyclobutylamino-äthanolhydrochlorid

Schmelzpunkt: 164-166°C (Zers.)

1-(4-Amino-3-cyan-5-fluor-phenyl)-2-isopropylamino-äthanol-hydrochlorid

Schmelzpunkt: 182-184°C (Zers.)

1-(4-Amino-3-cyan-5-fluor-phenyl)-2-tert.butylamino-äthanol-hydrochlorid

Schmelzpunkt: 242-243°C (Zers.)

1-(4-Amin-3-cyan-phenyl)-2-cyclobutylamino-äthanol-hydrobromid

Schmelzpunkt: ab 193°C (Zers.)

1-(4-Amino-3-cyan-phenyl)-2-tert.pentylamino-äthanol

Schmelzpunkt: 143°C

1-(4-Amino-3-chlor-5-cyan-phenyl)-2-sec.butylamino-äthanol-dihydrochlorid

Schmelzpunkt: 190-191°C

1-(4-Amino-3-chlor-5-cyan-phenyl)-2-tert.pentylamino-äthanol-hydrochlorid

Schmelzpunkt: 218-220°C (Zers.)

1-(4-Amino-3-chlor-5-cyan-phenyl)-2-cyclopentylamino-äthanol-hydrochlorid

Schmelzpunkt: 138-144°C
1-(4-Amino-3-trifluormethyl-phenyl)-2-tert.butylamino-äthanol-hydrochlorid

Schmelzpunkt: 172-174°C (Zers.)
1-(4-Amin-3-trifluormethyl-phenyl)-2-tert.pentylamino-äthanol-hydrobromid

Schmelzpunkt: 174-175°C (Zers.)
1-(4-Amino-3-chlor-5-trifluormethyl-phenyl)-2-isopropylamino-äthanol

Schmelzpunkt: 104-106°C
1-(4-Amino-3-chlor-5-trifluormethyl-phenyl)-2-tert.butylamino-äthanol-hydrochlorid

Schmelzpunkt: 205-207°C (Zers.)
1-(4-Amino-3-chlor-5-trifluormethyl-phenyl)-2-cyclobutylamino-äthanol-hydrochlorid

Schmelzpunkt: 177-178°C (Zers.)

1-(4-Amino-3-chlor-5-trifluormethyl-phenyl)-2-tert.pentylamino-äthanol-hydrochlorid

Schmelzpunkt; 176-178°C (Zers.)
1-(4-Äthoxycarbonyl-3,5-dichlor-phenyl)-2-tert.butylamino-äthanol-hydrochlorid

Schmelzpunkt: 230-232°C (Zers.)
1-(4-Amino-3,5-dichlor-phenyl)-2-tert.butylamino-äthanol-hydrochlorid

Schmelzpunkt: 174-175,5°C (Zers.)

Beispiel 1

Einmischung von 1-(4-Amino-3,5-dichlor-phenyl)-2-tert.butylamino-äthanol-hydrochlorid in Milchaustauschfutter

Zusammensetzung der Milchaustauschfutter:

| | |
|---|---|
| I. Masttag 1 - 60: | 18 % Fett<br>25 % Eiweiß |
| II. Masttag 61-120: | 21 % Fett<br>25 % Eiweiß |
| III. Masttag 121-160: | 23 % Fett<br>25 % Eiweiß |

Konzentrationen:

| | |
|---|---|
| Masttag 1-60 | 80-100 g/l bei 7 l/Tag |
| Masttag 61-120 | 120-140 g l bei 8 l/Tag |
| Masttag 121-160 | 220 g/l bei 8 l/Tag |

Für den empfohlenen Behandlungszeitraum von Tag 60-160 im Mastbestand beträgt die Dosis

a) bei Einmischen in das Milchaustauschfutter (MAF)

250 mg Wirksubstanz (Clenbuterol) pro t Milchaustauschfutter (MAF) = 0,25 ppm

MAF-Konzentration II:

| Körpergewicht (kg) | Erreichte Tagesdosis in mcg pro kg KGW bei Tagesration von | | | |
|---|---|---|---|---|
| | 960 g | 1040 g | 1120 g | 1760 g |
| 100 | 2,4 | 2,6 | 2,8 | |
| 110 | 2,2 | 2,4 | 2,5 | |
| 120 | 2,0 | 2,2 | 2,3 | |
| 130 | 1,8 | 2,0 | 2,2 | |
| 140 | 1,7 | 1,9 | 2,0 | |
| 150 | 1,6 | 1,7 | 1,9 | |
| 160 | 1,5 | 1,6 | 1,8 | |
| 170 | - | 1,5 | 1,6 | |
| 180 | - | - | 1,6 | 2,4 |

MAF-Konzentration III:

| Körpergewicht (kg) | Erreichte Tagesdosis in mcg pro kg KGW bei Tagesration von | | | |
|---|---|---|---|---|
| | 960 g | 1040 g | 1120 g | 1760 g |
| 190 | | | | 2,3 |
| 200 | | | | 2,2 |
| 210 | | | | 2,1 |
| 220 | | | | 2,0 |
| 230 | | | | 1,9 |
| 240 | | | | 1,8 |
| 250 | | | | 1,8 |
| 260 | | | | 1,7 |

b) bei Gabe der 1%igen Wirksubstanz-Lösung in das dem MAF zugesetzte Wasser und bei einer Tagesdosis von

2 mcg/kg Körpergewicht

pro 100 kg KGW = 0,2 ml Lösung

oder bei Berechnung auf die verwendete Wassermenge (8 l) pro Tier

| KGW in kg | Lösung in ml pro 8l Wasser | Dosis in ml pro 1l Wasser |
|---|---|---|
| 100 | 0,20 | 0,025 |
| 110 | 0,22 | 0,0275 |
| 120 | 0,24 | 0,03 |
| 130 | 0,26 | 0,0325 |
| 140 | 0,28 | 0,035 |
| 150 | 0,30 | 0,0375 |
| 160 | 0,32 | 0,04 |
| 170 | 0,34 | 0,0425 |
| 180 | 0,36 | 0,45 |
| 190 | 0,38 | 0,0475 |
| 200 | 0,40 | 0,05 |
| 210 | 0,42 | 0,0525 |
| 220 | 0,44 | 0,055 |
| 230 | 0,46 | 0,0575 |
| 240 | 0,48 | 0,06 |
| 250 | 0,50 | 0,0625 |
| 260 | 0,52 | 0,065 |

Beispiel 2

Einmischung von 1-(4-Amino-3,5-dichlor-phenyl)-2-tert.butylamino-äthanol-hydrochlorid in Milchaustauschfutter

Zusammensetzung des Milchaustauschfutter:

(für Mastkälber ab etwa 80 kg als Alleinfutter)

| | |
|---|---|
| Rohprotein: | mind. 17 % |
| Rohfett: | 15-30 % |
| Rohasche: | max. 10 % |
| Milchpulver: | mind. 25 % |

Für den empfohlenen Behandlungszeitraum ab 3. Mastwoche bis Mastende beträgt die Dosis

a) bei Einmischen in das Milchaustauschfutter (MAF)

100 mg Wirksubstanz (Clenbuterol)

pro t MAF = 0,1 ppm

BayWa Tränkeplan mit Biofix-Raiffeisen-Milchaustauscher

| Mastwoche | KGW bei Wochenbeginn | Tagesration | Erreichte Tagesdosis in mcg/kg KGW |
|---|---|---|---|
| 3. | 76 | 1,5 | 2,0 |
| 4. | 83 | 1,8 | 2,2 |
| 5. | 91 | 1,9 | 2,1 |
| 6. | 101 | 2,1 | 2,1 |
| 7. | 111 | 2,3 | 2,1 |
| 8. | 122 | 2,5 | 2,0 |
| 9. | 134 | 2,7 | 2,0 |
| 10. | 147 | 2,9 | 2,0 |

b) Bei Gabe der 1%igen Wirksubstanz-Lösung in das dem MAF zugesetzte Wasser und bei einer Tagesdosis von 2 mcg/kg KGW

pro kg KGW = 0,2 ml Lösung

oder bei Berechnung auf die verwendete Wassermenge pro Tier

| KGW bei Wochenbeginn in kg | Flüssigkeitsmenge | Dosis in ml/1l Wasser |
|---|---|---|
| 76 | 8 l | 0,019 |
| 83 | 9 l | 0,018 |
| 91 | 9 l | 0,02 |
| 101 | 10 l | 0,02 |
| 111 | 10 l | 0,022 |
| 122 | 10 l | 0,022 |
| 134 | 11 l | 0,024 |
| 147 | 11 l | 0,026 |

In den vorstehenden Beispielen können an Stelle von Clenbuterol selbstverständlich alle übrigen Verbindungen der allgemeinen Formel I in den erfindungsgemäßen Dosen eingesetzt werden.

**Ansprüche**

1. Verwendung einer Verbindung der allgemeinen Formel

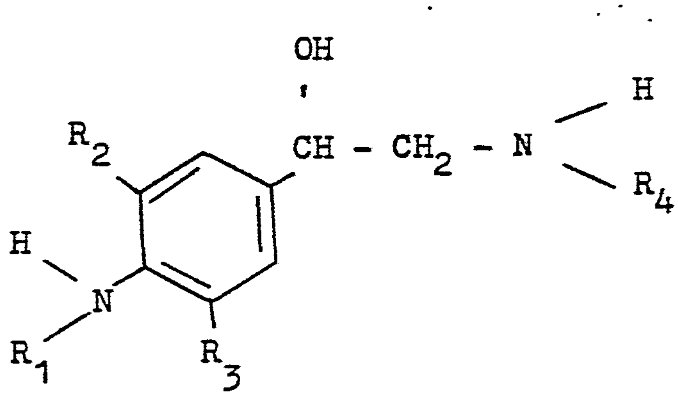

,(I)

in der

$R_1$ ein Wasserstoffatom oder eine Alkoxycarbonylgruppe mit insgesamt 2 bis 5, Kohlenstoffatomen,

$R_2$ ein Wasserstoff-, Fluor-oder Chloratom,

$R_3$ ein Chlor-oder Bromatom, eine Cyan-oder Trifluormethylgruppe und

$R_4$ eine verzweigte Alkylgruppe mit 3 bis 5 Kohlenstoffatomen oder eine Cycloalkylgruppe mit 3 bis 5 Kohlenstoffatomen bedeuten, und deren physiologisch verträgliche Säureadditionssalze zur Behandlung und Verhinderung peptischer Ulcera.

2. Verwendung einer Verbindung der allgemeinen Formel I gemäß Anspruch 1, in der

$R_1$ ein Wasserstoffatom oder eine Äthoxycarbonylgruppe,

$R_2$ ein Fluor-oder Chloratom,

$R_3$ ein Chloratom, eine Cyan-oder Trifluormethylgruppe und

$R_4$ eine verzweigte Alkylgruppe mit 3 bis 5 Kohlenstoffatomen bedeuten, und deren physiologisch verträgliche Säureadditionssalze zur Behandlung und Verhinderung peptischer Ulcera.

3. 1-(4-Amino-3,5-dichlor-phenyl)-2-tert.butylamino-äthanol und dessen physiologisch verträgliche

Säureadditionssalze zur Behandlung und Verhinderung peptischer Ulcera.

4. 1-(4-Amino-3-chlor-5-trifluormethyl-phenyl)-2-tert.butylamino-äthanol und dessen physiologisch verträgliche Säureadditionssalze zur Behandlung und Verhinderung peptischer Ulcera.

5. 1-(4-Äthoxycarbonylamino-3-cyan-5-fluor-phenyl)-2-tert.butyl-amino-äthanol und dessen physiologisch verträgliche Säureadditionssalze zur Behandlung und Verhinderung peptischer Ulcera.

6. Verwendung einer Verbindung gemäß den Ansprüchen 1 bis 5 als Futterzusatz zur Behandlung und Verhinderung peptischer Ulcera.

7. Verwendung einer Verbindung gemäß den Ansprüchen 1 bis 5 als Futterzusatz zur Behandlung und Verhinderung peptischer Ulcera bei der Protein-Masternährung.

8. Verwendung einer Verbindung gemäß den Ansprüchen 1 bis 5 zur Herstellung eines Futtermittels zur Behandlung und Verhinderung peptischer Ulcera.

9. Verfahren zur Herstellung eines Futtermittels zur Behandlung und Verhinderung peptischer Ulcera, dadurch gekennzeichnet, daß ein proteinhaltiges Futtermittel mit einer Verbindung gemäß den Ansprüchen 1 bis 5 in der Weise vermischt wird, daß die Tagesdosis einer Verbindung der allgemeinen Formel I 1 bis 10 mcg/kg Körpergewicht, vorzugsweise jedoch 1,5 bis 5 mcg/kg Körpergewicht, beträgt.

10. Verfahren gemäß Anspruch 9, dadurch gekennzeichnet, daß als proteinhaltiges Futtermittel Milchaustauscher verwendet wird.